# EUROPEAN PATENT APPLICATION

(11) **EP 4 186 499 A1**
(43) Date of publication of application: **31.05.2023**
(21) Application number: 22209184.5
(22) Date of filing: 23.11.2022
(51) Int. Cl.: A61K 31/194, A61K 31/198, A61K 31/401, A61K 31/728, A61P 27/02

(54) **OPHTHALMIC COMPOSITIONS FOR TREATMENT OF CORNEAL LESIONS**

(30) Priority: 24.11.2021 IT 202100029726
(71) Applicant: Professional Dietetics S.p.A., 20129 Milano (MI) (IT)
(72) Inventor: GIORGETTI, Paolo, 20129 MILANO (MI) (IT)
(74) Representative: Bianchetti & Minoja with Trevisan & Cuonzo IPS SRL

(57) **Abstract**

The present invention relates to ophthalmic compositions comprising a mixture of amino acids, hyaluronic acid, citric acid, succinic acid and malic acid.

## Description

The present invention relates to ophthalmic compositions comprising a mixture of amino acids, hyaluronic acid, citric acid, succinic acid and malic acid.

### PRIOR ART

Corneal disorders, which are very common disorders that affect up to 70% of diabetic patients examined, are due to peripheral neuropathy, the symptoms whereof include delayed wound healing, compromised barrier function, persistent epithelial defects and ulcers, recurrent erosions, epithelial oedema, reduced corneal sensitivity, neurotrophic corneal ulcers, and dysfunction of the corneal epithelial stem cells located in the limbal area wherein they play a crucial role in the maintenance of normal corneal homeostasis and in corneal wound healing.

Corneal wounds are still the most common form of eye damage. The majority of said lesions are abrasions of the corneal epithelium on the eye surface. Healing of a corneal wound is a complex process, and its mechanisms and the underlying genetic control are not fully understood. It involves the integrated actions of multiple growth factors, cytokines and proteases produced by epithelial cells, stromal keratocytes, inflammatory cells and tear gland cells. In corneal epithelial wound healing, the most common method of accelerating wound closure is to administer a topical medicament in the form of eyedrops. However, this approach has the drawback of low bioavailability, and requires repeated applications.

EP 1 940 381 discloses ophthalmic compositions comprising hyaluronic acid, glycine, proline, leucine and lysine, which are useful to perform a regenerating and protective action on the corneal epithelial cells.

EP 326 854 discloses compositions that stimulate biosynthesis of elastin and collagen, comprising hyaluronic acid, glycine, proline, leucine, valine, alanine and lysine in specific ratios. Said compositions are useful in the dermatological and osteoarticular fields.

WO 2019021135 discloses nutritional formulations comprising leucine, isoleucine, valine, threonine, lysine, citric acid, malic acid and succinic acid to improve the mitochondrial function, which are useful in particular for adjuvant treatment of sarcopenia and heart failure.

### DESCRIPTION OF THE INVENTION

It has now been found that a combination of tricarboxylic acid cycle intermediates (citric acid, malic acid and succinic acid) with a mixture of six amino acids (glycine, L-proline, L-alanine, L-valine, L-leucine and L-lysine hydrochloride) in specific ratios, carried in hyaluronic acid, is effective in the treatment of epithelial lesions of the cornea.

The object of the invention is therefore topical ophthalmic compositions comprising:
a) a mixture of amino acids consisting of glycine, L-proline, L-alanine, L-valine, L-leucine and L-lysine hydrochloride in the following weight ratios:
   - glycine 1;
   - L-proline: 0.7-0.8;
   - L-alanine: 0.47-0.76;
   - L-valine: 0.35-0.56;
   - L-leucine: 0.13-0.27;
   - L-lysine hydrochloride: 0.10-0.12:
b) citric acid, malic acid and succinic acid or salts thereof;
c) hyaluronic acid or salts thereof.

The preferred weight ratios of the amino acids are:
- glycine 1;
- L-proline: 0.75;
- L-alanine: 0.48-0.51;
- L-valine: 0.35-0.37;
- L-leucine: 0.13-0.15;
- L-lysine hydrochloride: 0.10-0.11.

More preferred weight ratios of the amino acids are:
- glycine 1;
- L-proline: 0.75;
- L-alanine: 0.75-0.76;
- L-valine: 0.54-0.56;
- L-leucine: 0.13-0.14;
- L-lysine hydrochloride: 0.10-0.11.

Most preferred weight ratios of the amino acids are:
- glycine 1;
- L-proline: 0.75;
- L-alanine: 0.75-0.76;
- L-valine: 0.54-0.56;
- L-leucine: 0.13-0.14;
- L-lysine hydrochloride: 0.10-0.11.

The weight ratios of citric acid, malic acid and succinic acid (CMS) are preferably 4:1:1.

The mixture of amino acids and tricarboxylic acid cycle intermediates in the ratios specified above is present in the compositions according to the invention in percentages ranging from 1 to 20%, preferably 2 to 10%, and more preferably 3 to 5% by weight.

The CMS mixture in the ratio of 4:1:1 can constitute 1 to 40% of the total mixture.

The hyaluronic acid or salts thereof, in particular sodium hyaluronate, can have an average molecular weight Mₙ ranging between 500,000 and 3,000,000 Da. The percentages of hyaluronic acid or sodium hyaluronate range between 0.01 and 3% by weight of the total composition.

The compositions according to the invention can, for example, take the form of eyedrops, ointments, gels or sterile solutions.

The compositions according to the invention will be used, particularly, for the treatment of:
- mild, moderate or serious alterations of the tear film, with a reduction in the typical symptoms of dry eye.
- patients who undergo laser treatments (PRK): the reduction in post-operative re-epithelialisation time prevents the appearance of haze and dry eye syndrome which are typical of the first few months after surgery;
- relapsing and/or persistent corneal ulcers: the lasting re-epithelialisation effect prevents relapses and allows complete re-epithelialisation;
- cataract removal surgery, phacoemulsification: the rapid healing effect on the corneal tunnel significantly reduces the discomfort felt by the patient.

The compositions according to the invention will be applied to the eye 2-6 times a day, until the disorder improves or is cured.

The activity of the compositions according to the invention has been demonstrated with TKE2 cells, a progenitor cell line deriving from murine limbal/corneal epithelium, evaluating the gene expression of HA, succinate and citrate carriers/receptors (RHAMM, SCNR1 and Scl25a1 respectively). The protein levels of PGC-1α, Cyt c and ITGB1 were also analysed. The compositions increased mitochondrial biogenesis markers, FN1 and ITGB1 expression in corneal TKE2 cells.

Correct maintenance of mitochondrial homeostasis is essential for cell health. Stimulation of mitochondrial biogenesis represents a valuable therapeutic tool for the prevention and treatment of disorders characterised by an energy metabolism deficiency.

The most significant experiments conducted are described by way of example.

The mRNA levels of the HA-mediated motility receptor (RHAMM), succinate receptor (SUCNR1) and citrate membrane carrier (Scl25a1) were measured in TKE2 cells treated with the two mixtures of four amino acids (EP 1 940 381) or the six amino acids specified above, with or without CMS, for 24 h.

### Cell culture and treatment

The TKE2 cells were cultured in a medium defined as keratinocyte serum-free (Stemline Keratinocyte Medium II, Sigma-S0196, Italy) supplemented with Stemline Keratinocyte Growth Supplement (Sigma-S9945, Italy), supplied by the manufacturer until the time of use. The cell cultures were incubated at 37°C, with 95% humidity and 5% CO₂. The cells were treated with 0.01%, 0.1%, 0.5% or 1% of the amino-acid mixtures (composition shown in Table 1) for 48 hours or 24 hours. The untreated cells were plated as controls. Every 24 h, the media were replaced in both the control flasks and the treatment flasks with fresh media or amino-acid mixtures with or without the addition of a 5 mM solution of citric, malic and succinic acids in the ratio of 8:2:2 respectively. At the end of the experimental treatments, the cells were used for mRNA extraction, protein level analysis, cell viability evaluation and the in-vitro graft damage model.

**Table 1. Percentage composition by weight of amino-acid mixtures**

| **Mixture** | **4AA (%)** | **6AA (%)** |
|---|---|---|
| L-leucine | 5.38 | **3.23** |
| L-lysine (hydrochloride) | 4.23 | **2.54** |
| L-valine | - | **12.92** |
| Glycine | 38.46 | **23.23** |
| L-proline | 28.85 | **17.46** |
| L-alanine | - | **17.54** |
| Sodium hyaluronate | 23.07 | **23.07** |

### Total RNA extraction and gene expression analysis

RNA was isolated from the TKE2 cells with the RNeasy Mini Kit (Qiagen), and cDNA was synthesised with the iScript cDNA Synthesis Kit (Bio-Rad Laboratories). The quantitative RT-PCRs were performed with the iQ SybrGreenI SuperMix (Bio-Rad; Segrate, Italy) on the iCycler iQ real-time PCR detection system (Bio-Rad). Briefly, RNA was isolated from the TKE2 cells with the RNeasy Tissue Mini Kit (Qiagen, Segrate, Italy). cDNA was synthesised with the iScript cDNA Synthesis Kit (Bio-Rad Laboratories). The primers were designed with Beacon Designer 2.6 software from Premier Biosoft International (Palo Alto, CA, USA), and are shown in Table 2.

**Table 2. qRT-PCR primers**

| **Gene** | **Sense Primer (5'-3')** | **Antisense Primer (5'-3')** | **PCR Product (bp)** | **Ta (°C)** |
|---|---|---|---|---|
| ***RHAMM*** | CCTTGCTTGCTTCGGCT AAAA | CTGCTGCATTGAGC TTTGCT | 190 | 60 |
| ***Scl25a1*** | TCGGGAACAAGGGCTA AAGG | CCCGTGATCAGTG GGTTCAT | 157 | 60 |
| ***SUCNR1*** | ACAGAAGCCGACAGCA GAAT | GCACAGGAAAGCA AAGTCAG | 229 | 50 |

The number of cycles wherein the various transcriptions were detectable was compared (threshold cycle, CT) with TBP, including as ΔCT. The corresponding gene level was expressed as 2-(ΔΔCT), wherein ΔΔCT is equal to the difference between the DCT of the treated TKE2 cells and the ΔCT of the untreated TKE2 cells.

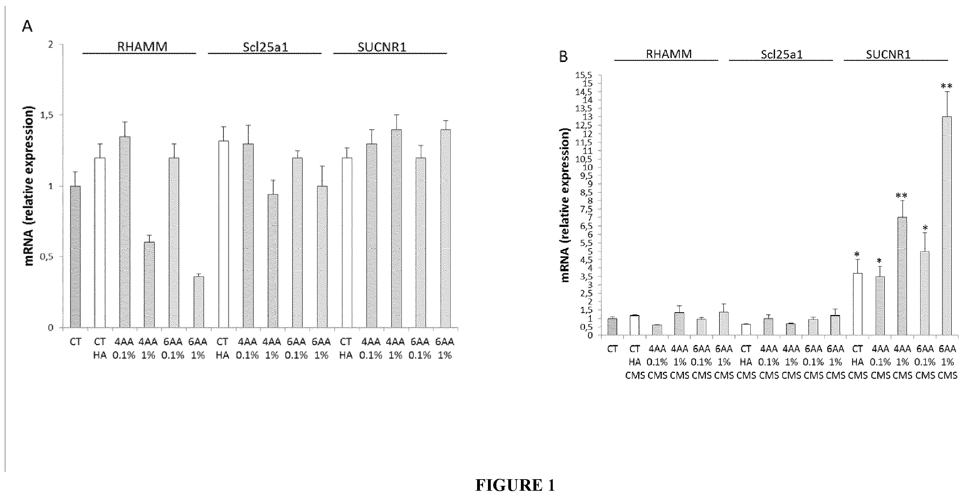

As shown in Figure 1A, the RHAMM, SUCNR1 and Scl25a1 mRNA levels exhibited a tendency to increase, compared with the levels in the untreated TKE2 cells. The addition of a 5mM aqueous solution of citric, malic and succinic acids (CMS) in the ratio of 8:2:2 significantly increased the mRNA levels of SUCNR1, to a greater extent than the 4 amino acids (4AA) described in EP 1 940 381, or the 6 amino acids (6AA).

Figure 1 shows the mRNA levels of RHAMM, SUCNR1 and Scl25a1 in TKE2 fibroblasts treated with 0.1-1% 4AA or 6AA for 24 h (A) and more CMS (B). A quantitative PCR was used, performed in triplicate and normalised to TBP (n = 3, mean ± SEM). * P < 0.05 and ** P < 0.01 vs untreated cells, expressed as 1.0. The results demonstrate that enrichment of the six amino acids with CMS promotes favourable effects in corneal epithelial cells.

As it has been demonstrated that SUCNR1 levels increase in the presence of hypoxia, and that stimulation of the succinate receptors in the eyes gives rise to production of vascular epithelial growth factor A, supporting vascularisation after the lesion, the increase in SUCNR1 is desirable for the purpose of regeneration processes after the injury.

The findings are plausibly predictive of the clinical usefulness of the formulations according to the invention, as also confirmed by Favret et al., Aging, 2013, Vol. 5 N. 6-427-444, who discuss the pathogenetic role of a SUCNR1 receptor deficiency in retinal lesions.

## Claims

1. Topical ophthalmic compositions comprising:
a) an amino-acid mixture consisting of glycine, L-proline, L-alanine, L-valine, L-leucine and L-lysine hydrochloride in the following weight ratios:
- Glycine 1;
- L-Proline: 0.7-0.8;
- L-Alanine: 0.47-0.76;
- L-Valine: 0.35-0.56;
- L-Leucine: 0.13-0.27;
- L-Lysine hydrochloride: 0.10-0.12.
b) citric acid, malic acid and succinic acid or salts thereof;
c) hyaluronic acid or salts thereof.

2. Compositions according to claim 1 wherein the weight ratios of the amino acids are:
- Glycine 1;
- L-Proline: 0.75
- L-Alanine: 0.48-0.51;
- L-Valine: 0.35-0.37;
- L-Leucine: 0.13-0.15;
- L-Lysine hydrochloride: 0.10-0.11.

3. Compositions according to claim 1 wherein the weight ratios of the amino acids are:
- Glycine 1;
- L-Proline: 0.75
- L-Alanine: 0.75-0.76;
- L-Valine: 0.54-0.56;
- L-Leucine: 0.13-0.14;
- L-Lysine hydrochloride: 0.10-0.11.

4. Compositions according to claim 1 wherein the weight ratios of the amino acids are:
- Glycine 1;
- L-Proline: 0.75
- L-Alanine: 0.75-0.76;
- L-Valine: 0.54-0.56;
- L-Leucine: 0.13-0.14;
- L-Lysine hydrochloride: 0.10-0.11.

5. Compositions according to any one of claims 1 to 4 wherein the weight ratios of citric acid, malic acid and succinic acid are 4:1:1.

6. Compositions according to any one of claims 1 to 5 wherein the hyaluronic acid or salts thereof, particularly sodium hyaluronate, has an average molecular weight ranging between 500,000 and 3,000,000 Da, in percentages ranging between 0.01 and 3% by weight of the total composition.

7. Compositions according to any one of claims 1 to 6 in the form of eyedrops, ointments, gels or sterile solutions.

8. The compositions of claims 1-7 for use in the treatment of corneal injuries.
